# EUROPÄISCHE PATENTSCHRIFT

(11) **EP 0 803 494 B1**
(45) Veröffentlichungstag und Bekanntmachung des Hinweises auf die Patenterteilung: **21.03.2001**
(21) Anmeldenummer: 97100724.0
(22) Anmeldetag: 17.01.1997
(51) Int. Cl.: C07C 67/54, C07C 69/82

(54) **Verfahren zur Destillation von Rohester im DMT/PTA-Verfahren**
Process for destillation of crude ester in the DMT/PTA process
Procédé de distillation d'esters bruts dans le procédé DMT/PTA

(30) Priorität: 22.04.1996 DE 19615886
(43) Veröffentlichungstag der Anmeldung: 29.10.1997
(73) Patentinhaber: Degussa-Hüls Aktiengesellschaft, 60287 Frankfurt am Main (DE)
(72) Erfinder: Lenz, Udo, 45657 Recklinghausen (DE); Neutzler, Ulrich, 58300 Wetter (DE); Schoengen, Anton, 58452 Witten (DE); Sigg, Reinhard, Dr., 45772 Marl (DE)

(56) Entgegenhaltungen:
- WO-A-90/09367
- DE-A- 3 011 858
- Ullmann's Encyclopedia of Industrial Chemistry, Bd. 26, 1995, S. 193-200
- Reinhard Billet, Packed Towers in Processing and Environmental Technology, Weinheim 1995, insbesondere S. 25-27, 255-262, 96-100, 238-240
- Klaus Sattler, Thermische Trennverfahren, Grundlagen, Auslegung, Apparate, Weinheim 1995, insbesondere S. 232-237

## Beschreibung

Die Erfindung betrifft ein Verfahren zur Herstellung von rohem Dimethylterephthalat (Roh-DMT), das im wesentlichen folgende Verfahrensschritte beinhaltet,
- Oxidation von para-Xylol (p-X) und para-Toluylsäuremethylester (p-TE),
- Veresterung der Reaktionsprodukte aus der Oxidation mit Methanol,
- Trennung des entstandenen, sogenannten Rohesters in
   a) eine Fraktion, die in die Oxidation zurückgeführt wird,
   b) eine Roh-DMT-Fraktion und
   c) eine schwersiedende Rückstandsfraktion.

Dimethylterephthalat (DMT) und Terephthalsäure (TA) werden großtechnisch in zahlreichen Anlagen weltweit hergestellt. DMT und TA sind wichtige Ausgangsverbindungen für die Herstellung von Polyestem. Die Anwendungsgebiete der Polyester für Fasern sowie Folien, u. a. für fotographische Filme und Magnetbänder oder Flaschen aus Polyethylenterephthalat, um nur einige zu nennen, sind lange bekannt.

Es ist bekannt, daß das heutige Witten-DMT-Verfahren im wesentlichen die Verfahrensschritte
- Oxidation von para-Xylol (p-X) und para-Toluylsäuremethylester (p-TE), in der Regel mit nachgeschalteter Abgasreinigung,
- Veresterung der Reaktionsprodukte aus der Oxidation mit Methanol,
- Trennung des entstandenen, sogenannten Rohesters in
   a) eine Fraktion, die in die Oxidation zurückgeführt wird,
   b) eine Roh-DMT-Fraktion, mit mehr als 85 Gew.-% DMT und
   c) eine schwersiedende Rückstandsfraktion, gegebenenfalls deren Aufarbeitung, beispielsweise durch Methanolyse oder Thermolyse, und eine nachfolgende Rückgewinnung des Katalysators,
- Reinigung der Roh-DMT-Fraktion, beispielsweise durch Waschen, Umkristallisieren und Reindestillation, beinhaltet ("Terephthalsäuredimethylester", Ullmann Bd. 22, 4. Auflage, S. 529 - 533; EP 0 464 046 B1; DE-OS 40 26 733). Es ist auch möglich aus Roh-DMT-Fraktionen, die besonders reich an DMT sind, oder aus DMT-reinst durch eine gezielte Hydrolyse Terephthalsäure entsprechender Qualität herzustellen.

Die Oxidation eines Gemisches aus para-Xylol (p-X) und para-Toluylsäuremethylester (p-TE oder pT-Ester) wird im allgemeinen mit Luftsauerstoff in Gegenwart eines Schwermetallkatalysators (DE-PS 20 10 137) bei einer Temperatur von etwa 140 bis 180 °C und einem Druck von etwa 4 bis 8 bar abs. in flüssiger Phase durchgeführt. Aus der Oxidationsstufe resultiert ein Reaktionsgemisch, das überwiegend Monomethylterephthalat (MMT), p-Toluylsäure (p-TA) und Terephthalsäure (TA), gelöst bzw. suspendiert in p-TE, enthält und mit Methanol bei einer Temperatur von etwa 250 bis 280 °C und einem Druck von 20 bis 25 bar abs. verestert wird. Der erhaltene Rohester wird destillativ in eine p-TE-Fraktion, eine Roh-DMT-Fraktion und eine hochsiedende, Katalysator-haltige Rückstandsfraktion aufgetrennt. Die p-TE-Fraktion wird in die Oxidation zurückgeführt und die Roh-DMT-Fraktion über nachfolgende Reinigungsstufen in die gewünschte Produktqualität überführt. Die aus der Rohester-Destillation stammende Rückstandsfraktion wird im allgemeinen einer Methanolyse oder einer Thermolyse unterzogen. Auch sind Verfahren zur Rückgewinnung und Wiederverwertung des Schwermetalloxidationskatalysators aus den hochsiedenden und katalysatorhaltigen Rückständen, wie sie im allgemeinen bei der Oxidation, Veresterung oder der Rohester-Destillation entstehen, bekannt.

Die DE-OS 30 11 858 offenbart ein Verfahren zur Rohester-Destillation mit einem 3-Kolonnensystem und lehrt ferner, daß für die TAE-Destillation eine druckverlustarme Kolonne besonders geeignet ist, z. B. solche mit geeigneten Packungen oder Füllkörpern. Solche 3-Kolonnensysteme erfordern ein hohes Investment.

Füllkörper und Packungen sind im allgemeinen aus "Reinhard Billet, Packed Towers in Processing and Enviromental Technology, Weinheim 1995, Seiten 25 bis 27, 96 bis 100, 238 bis 240 und 255 bis 262," oder aus "Klaus Sattler, Thermische Trennverfahren, Grundlagen Auslegung, Apparate, Weinheim 1995, Seiten 232 bis 237, bekannt.

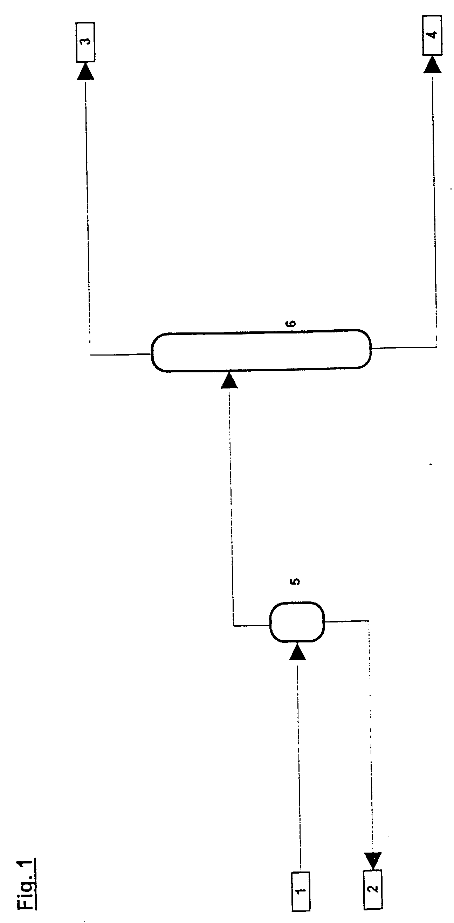

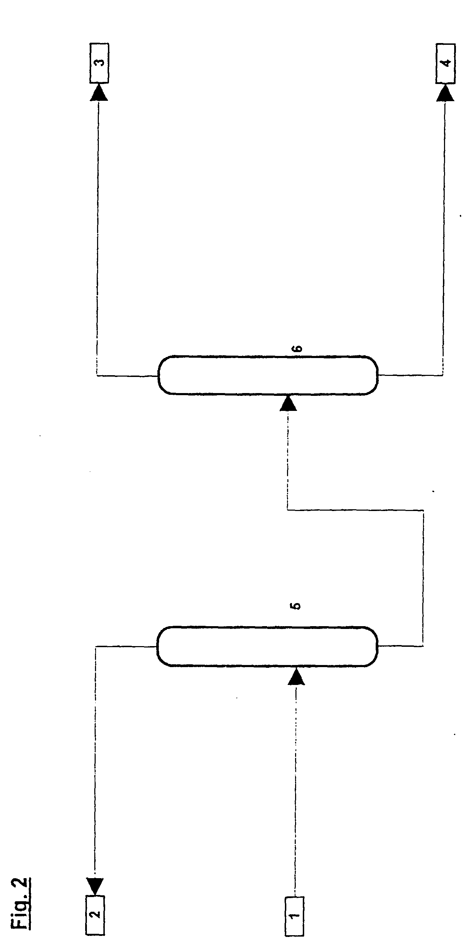

Beim Witten-DMT-Verfahren sind als Destillations-Einheiten für die Durchführung der Rohester-Destillation im allgemeinen das sogenannte Flashkammersystem (fig. 1) sowie das 2-Kolonnensystem (Fig. 2) im Einsatz. Die Kolonnen dieser Systeme sind üblicherweise als Bodenkolonnen ausgeführt. Die Rohester-Destillation wird unter vermindertem Druck (Vakuum) betrieben, wobei üblicherweise an den Kolonnenköpfen Drücke zwischen 20 und 200 mbar eingestellt werden. Aus diesen Betriebsbedingungen resultieren relativ hohe Sumpftemperaturen, die in der Regel im Bereich von 230 bis 270 °C liegen. Es ist von Nachteil, daß durch hohe Sumpftemperaturen verstärkt unerwünschte Nebenreaktionen, beispielsweise die Bildung sogenannter Hochsieder, auftreten. Durch eine Absenkung des Druckes in der Rohester-Destillation könnte zwar eine Senkung der Betriebstemperatur in den Kolonnensümpfen erzielt werden, jedoch treten dann andere Nachteile auf: Aus einer Erhöhung der Vakua würden bei konstanter Anlagenkapazität höhere Gasvolumina resultieren, Kolonnen mit entsprechend größeren Kolonnendurchmessern würden erforderlich und somit auch ein höheres Investment. Ferner würde durch eine eventuelle Senkung der Kopfdrücke niedrigere Kondensationstemperaturen der am Kopf der Kolonnen anfallenden Destillationsbrüden resultieren. Üblicherweise werden die an dieser Stelle anfallenden Kondensationswärmen zur Erzeugung von Nieder- bzw. Mitteldruck-Dampf eingesetzt. Würde über die Druckabsenkung in den Kolonnenköpfen die Kondensationstemperatur erheblich abgesenkt, kann die Erzeugung von Dampf unwirtschaftlich bzw. unmöglich werden (Kondensationstemperatur < 100 °C). Dies wäre für die Energiebilanz über den gesamten Prozeß von Nachteil.

Der Erfindung lag die Aufgabe zugrunde, ein Verfahren bereitzustellen, das es ermöglicht, die Rohester-Destillation in möglichst einfacher und wirtschaftlicher Weise zu betreiben.

Die gestellte Aufgabe wird erfindungsgemäß entsprechend den Angaben der Patentansprüche gelöst.

Es wurde nun gefunden, daß unter Verwendung von Strukturpackungen, die im wesentlichen keine horizontal orientierten Strukturelemente enthalten, als Trenneinheiten und einer schonenderen Fahrweise in der Rohester-Destillation, insbesondere durch eine niedrigere Sumpftemperatur, höhere Ausbeuten und damit eine weitere Verbesserung der Wirtschaftlichkeit des Witten-DMT-Prozesses erzielbar ist. Durch die vorliegende Erfindung werden Ausbeutegewinne von > 1 %, bezogen auf die destillierte DMT-Menge im Vergleich zu bekannten Betriebsweisen, erzielt.

Gegenstand der vorliegenden Erfindung ist somit ein Verfahren zur Herstellung von rohem Dimethylterephthalat (Roh-DMT), das im wesentlichen folgende Verfahrensschritte beinhaltet,
- Oxidation von para-Xylol (p-X) und para-Toluylsäuremethylester (p-TE),
- Veresterung der Reaktionsprodukte aus der Oxidation mit Methanol,
- Trennung des entstandenen, sogenannten Rohesters in
   a) eine Fraktion, die in die Oxidation zurückgeführt wird,
   b) eine Roh-DMT-Fraktion und
   c) eine schwersiedende Rückstandsfraktion,
wobei man die Trennung des Rohesters in Destillations-Einheiten durchführt, die bei einer Kopftemperatur im Bereich von 100 bis 220 ° C bei Drücken von 30 bis 200 mbar und einer Sumpftemperatur im Bereich von 180 bis 260 °C betrieben werden, das dadurch gekennzeichnet ist, daß die Einheiten der Rohester-Destillation mit Strukturpackungen ausgerüstet sind, welche im wesentlichen keine horizontal orientierten Strukturelemente enthalten, und die jeweilige Druckdifferenz zwischen Kolonnenkopf und Kolonnensumpf 30 mbar nicht übersteigt.

Vorzugsweise betreibt man beim erfindungsgemäßen Verfahren die Destillations-Einheiten bei einer Kopftemperatur im Bereich von 100 bis 220 ° C bei Drücken von 30 bis 200 mbar und einer Sumpftemperatur im Bereich von 180 bis 245 °C, besonders vorzugsweise bei einer Sumpftemperatur im Bereich von 190 bis 230 °C.

Beim erfindungsgemäßen Verfahren soll die jeweilige Druckdifferenz zwischen Kolonnenkopf und Kolonnensumpf vorzugsweise 20 mbar, besonders vorzugsweise 15 mbar, nicht übersteigen.

Vorzugsweise führt man beim erfindungsgemäßen Verfahren die Rohester-Destillation in einem 2-Kolonnensystem oder in einem Flashkammersystem durch.

Unter Strukturpackungen sind beim erfindungsgemäßen Verfahren geordnete Trenneinheiten zu verstehen, bei denen die wechselnde Anordnung der einzelnen, schrägverzahnten Strukturelemente, wie z. B. Lamellen, offene, sich kreuzende Kanäle bilden. Solche Strukturelemente können z. B. geformte sowie ggf. auch durchbrochene Bleche oder Platten, Streckmetall, Drahtgewebe, Formteile aus Keramik- oder Kunststoff sein. Solche speziellen Strukturpackungen werden u. a. von der Firma SULZER AG, Schweiz, angeboten.

Geeigneterweise setzt man beim erfindungsgemäßen Verfahren Strukturpackungen aus nichtrostenden Stählen oder anderen korrosionsbeständigen Materialien ein - insbesondere Strukturpackungen aus Aluminium oder Hastelloy oder Titan oder Kunststoff, beispielsweise Teflon, oder Keramik, beispielsweise auf M-Silikat-Basis.

Insbesondere eignen sich für den Einsatz beim erfindungsgemäßen Verfahren solche Strukturpackungen, die eine Oberfläche von 100 bis 800 m²/m³ aufweisen.

Bei der vorliegenden Erfindung sind unter Strukturpackungen solche Strukturpackungen zu verstehen, die im wesentlichen keine horizontal orientierten Strukturelemente enthalten. Der im Rohester enthaltene Katalysator neigt im allgemeinen zur Auskristallisation und zur Feststoffbildung, dies betrifft auch die bekannten Systeme zur Rohester-Destillation. Durch die Vermeidung vieler horizontaler Flächen kann beim erfindungsgemäßen Verfahren die Bildung und das Absetzen von Ablagerungen, die zur Verstopfung und somit zum Ausfall der gesamten Rohester-Destillation führen können, vermieden werden. Beim erfindungsgemäßen Verfahren setzt man somit vorzugsweise Strukturpackungen mit möglichst geringen Flüssigkeits-hold-up ein. Das ist ein deutlicher Vorteil gegenüber Packungen aus Füllkörpern und Ventilböden bekannter Art.

Darüber hinaus ist es beim erfindungsgemäßen Verfahren von Vorteil, wenn man Strukturpackungen einsetzt, die vorzugsweise mit einer Oberfläche ausgestattet sind, die Ablagerungen sowie Anhaftungen vermeidet. Hierzu können geeigneterweise besonders glatte Oberflächen bzw. Materialien eingesetzt werden, beispielsweise Teflon oder Materialien, die in ihrer diesbezüglichen Wirkung dem Teflon ähnlich sind.

Die Maßnahmen des erfindungsgemäßen Verfahrens bewirken somit den Vorteil, daß bei vorgegebenen Kopftemperaturen in der Rohester-Destillation eine noch produktschonendere und eine noch wirtschaftlichere Fahrweise ermöglicht wird.

Neben einer deutlichen Verbesserung der Wirtschaftlichkeit ist beim erfindungsgemäßen Verfahren auch festzustellen, daß die Neigung zu Ablagerungen in der Rohester-Destillation geringer ist als bei den bisherigen Verfahren.

Die Erfindung wird durch das nachfolgende Beispiel näher erläutert:

### Beispiel:

Die Betriebspraxis zeigt, daß bei einer gegebenen Verweilzeit und einer Sumpftemperatur von ca. 260 °C in der Flashkammer, vgl. Fig. 1, ca. 8,6 % Hochsieder, bezogen auf die destillierte DMT-Menge, neu gebildet werden und nach bekannten Verfahren günstigstenfalls 50 % der gebildeten Hochsieder wieder in Wertprodukte (z. B. in einer Methanolyse) zurückgeführt werden können. Der Ausbeuteverlust beträgt hier somit rd. 4,3 %.

In einem Versuch wurde nun der Rohester nach Einbau der Strukturpackung bei einer Sumpftemperatur von 190 °C unter schonenden Bedingungen destilliert. Bei dem

Versuch betrugen die Verluste durch Hochsiederbildung, bezogen auf das im Rohester enthaltene DMT, lediglich 5,4 %. Bei einer Rückgewinnungsrate von 50 % verbleibt nur noch ein Ausbeuteverlust von 2,7 %.

Durch den Einbau der Strukturpackung und die geringere Sumpftemperatur bei der Rohester-Destillation wurde so die Wirtschaftlichkeit des Prozesses deutlich verbessert. Dies entspricht einer Ausbeutesteigerung im Vergleich zur bekannten Fahrweise der Flashkammer von 1,6 %, bezogen auf die destillierte DMT-Menge im Rohester.

### Legende zu Fig. 1

Figur 1 zeigt ein Blockschema einer Rohester-Destillation im DMT-Prozeß, das als Flashkammersystem bezeichnet wird:

### Stoffströme -

- **1**: Rohester aus der Veresterung
- **2**: Hochsiedende, Katalysator-haltige Rückstandsfraktion zur Aufarbeitung
- **3**: Rückführung der p-TE-Fraktion in die Oxidation
- **4**: Roh-DMT zur weiteren Reinigung

### Anlagenteile -

- **5**: Flashkammer
- **6**: Rohester-Destillationskolonne

### Legende zu Fig. 2

Figur 2 zeigt ein Blockschema einer Rohester-Destillation im DMT-Prozeß, die als Zwei-Kolonnen-System bezeichnet wird:

### Stoffströme -

- **1**: Rohester aus der Veresterung
- **2**: Rückführung der p-TE-Fraktion in die Oxidation
- **3**: Roh-DMT-Fraktion zur weiteren Reinigung
- **4**: Hochsiedende, Katalysator-haltige Rückstandsfraktion zur Aufarbeitung

### Anlagenteile -

- **5**: p-TE-Destillationskolonne
- **6**: Roh-DMT-Destillationskolonne

### Legende der Abkürzungen:

- p-X :: para-Xylol
- p-TA :: para-Toluylsäure
- p-TE :: para-Toluylsäuremethylester (pT-Ester)
- BME :: Benzoesäuremethylester
- HM-BME :: Hydroxymethylbenzoesäuremethylester
- MM-BME :: Methoxymethylbenzoesäuremethylester
- DMT :: Dimethylterephthalat
- DMT-roh =: Rohester (DMT-Rohesterstrom nach der Veresterung)
- Roh-DMT :: Dimethylterephthalatfraktion nach der Rohester-Destillation
- DMT-reinst :: Dimethylterephthalat-reinst (hochreines DMT-Zwischen- oder Endprodukt)
- DMO :: Dimethylorthophthalsäure
- DMI :: Dimethylisophthalsäure
- DMP :: Dimethylphthalate = Isomerengemisch aus DMT, DMO und DMI
- MMT :: Monomethylterephthalat (Terephthalsäuremonomethylester)
- TA :: Terephthalsäure
- MTA :: mittelreine Terephthalsäure
- PTA :: Terephthalsäure hoher Reinheit
- PTA-p :: Terephthalsäure sehr hoher, d. h. höchster Reinheit (Gehalt an MMT und p-TA von zusammen < 50 Gew.-ppm)
- TAS :: Terephthalaldehydsäure (4-CBA)
- TAE :: Terephthalaldehydsäuremethylester

## Patentansprüche

1. Ein Verfahren zur Herstellung von rohem Dimethylterephthalat (Roh-DMT), das im wesentlichen folgende Verfahrensschritte beinhaltet,
- Oxidation von para-Xylol (p-X) und para-Toluylsäuremethylester (p-TE),
- Veresterung der Reaktionsprodukte aus der Oxidation mit Methanol,
- Trennung des entstandenen, sogenannten Rohesters in
a) eine Fraktion, die in die Oxidation zurückgeführt wird,
b) eine Roh-DMT-Fraktion und
c) eine schwersiedende Rückstandsfraktion,
wobei man die Trennung des Rohesters in Destillations-Einheiten durchführt, die bei einer Kopftemperatur im Bereich von 100 bis 220 ° C bei Drücken von 30 bis 200 mbar und einer Sumpftemperatur im Bereich von 180 bis 260 °C betrieben werden,
dadurch gekennzeichnet,
daß die Einheiten der Rohester-Destillation mit Strukturpackungen ausgerüstet sind, welche im wesentlichen keine horizontal orientierten Strukturelemente enthalten, und die jeweilige Druckdifferenz zwischen Kolonnenkopf und Kolonnensumpf 30 mbar nicht übersteigt.

2. Verfahren nach Anspruch 1,
dadurch gekennzeichnet,
daß man die Destillations-Einheiten bei einer Kopftemperatur im Bereich von 100 bis 220 °C bei Drücken von 30 bis 200 mbar und einer Sumpftemperatur im Bereich von 180 bis 245 °C betreibt.

3. Verfahren nach Anspruch 2,
dadurch gekennzeichnet,
daß man die Destillations-Einheiten bei einer Kopftemperatur im Bereich von 100 bis 220 °C bei Drücken von 30 bis 200 mbar und einer Sumpftemperatur im Bereich von 190 bis 230 °C betreibt.

4. Verfahren nach mindestens einem der Ansprüche 1 bis 3,
dadurch gekennzeichnet,
daß die jeweilige Druckdifferenz zwischen Kolonnenkopf und Kolonnensumpf 20 mbar nicht übersteigt.

5. Verfahren nach Anspruch 4,
dadurch gekennzeichnet,
daß die jeweilige Druckdifferenz zwischen Kolonnenkopf und Kolonnensumpf 15 mbar nicht übersteigt.

6. Verfahren nach mindestens einem der Ansprüche 1 bis 5,
dadurch gekenzeichnet,
daß die Rohester-Destillation in einem 2-Kolonnensystem durchgeführt wird.

7. Verfahren nach mindestens einem der Ansprüche 1 bis 6,
dadurch gekennzeichnet,
daß die Rohester-Destillation in einem System durchgeführt wird, das im wesentlichen aus einer Flashkammer und einer nachgeschalteten Kolonne besteht.

8. Verfahren nach mindestens einem der Ansprüche 1 bis 7,
dadurch gekennzeichnet,
daß man Strukturpackungen aus nichtrostenden Stählen oder anderen korrosionsbeständigen Materialien einsetzt.

9. Verfahren nach Anspruch 8,
dadurch gekennzeichnet,
daß man Strukturpackungen aus Aluminum oder Hostelloy oder Titan oder Kunststoff oder Keramik einsetzt.

10. Verfahren nach mindestens einem der Ansprüche 1 bis 9,
dadurch gekennzeichnet,
daß man Strukturpackungen mit einer Oberfläche von 100 bis 800 m²/m³ einsetzt.

## Claims

1. A process for the preparation of crude dimethyl terephthalate (crude DMT), which essentially comprises the following process steps.
- oxidation of para-xylene (p-X) and methyl paratoluate (p-TE),
- esterification with methanol of the reaction products from the oxidation,
- separation of the resulting so-called crude ester into
a) a fraction which is recycled to the oxidation,
b) a crude DMT fraction and
c) a high boiling residue fraction,
the separation of the crude ester being carried out in distillation units which are operated at an overhead temperature in the range from 100 to 220°C at pressures of from 30 to 200 mbar and a bottom temperature in the range from 180 to 260°C,
characterized in that the crude ester distillation units are equipped with structured packings which essentially contain no horizontally orientated structural elements, and the respective pressure difference between column top and column bottom does not exceed 30 mbar.

2. A process according to claim 1, characterized in that the distillation units are operated at an overhead temperature in the range from 100 to 220°C at pressures of from 30 to 200 mbar and at a bottom temperature in the range from 180 to 245°C.

3. A process according to claim 2, characterized in that the distillation units are operated at an overhead temperature in the range from 100 to 220°C at pressures of from 30 to 200 mbar and at a bottom temperature in the range from 190 to 230°C.

4. A process according to at least one of claims 1 to 3, characterized in that the respective pressure difference between column top and column bottom does not exceed 20 mbar.

5. A process according to claim 4, characterized in that the respective pressure difference between column top and column bottom does not exceed 15 mbar.

6. A process according to at least one of claims 1 to 5, characterized in that the crude ester is distilled in a two-column system.

7. A process according to at least one of claims 1 to 6, characterized in that the crude ester is distilled in a system which essentially comprises a flash chamber and a downstream column.

8. A process according to at least one of claims 1 to 7, characterized in that structured packings of stainless steels or other corrosion-resistant materials are used.

9. A process according to claim 8, characterized in that structured packings of aluminium or Hastelloy or titanium or plastic or ceramic are used.

10. A process according to at least one of claims 1 to 9, characterized in that structured packings having a surface area of from 100 to 800 m²/m³ are used.

## Revendications

1. Procédé de production de téréphtaLAte de diméthyle brut (DMT brut) qui comprend essentiellement les étapes suivantes de procédé :
• oxydation du paraxylène (p-X) et d'ester méthylique de l'acide para-toluylique (p-TE)
• estérification des produits de réaction provenant de l'oxydation, avec du méthanol,
• fractionnement de l'ester dénommé « ester brut », qui s'est formé, en
a) une fraction qui est ramenée à l'oxydation,
b) une fraction du DMT brut, et
c) une fraction résiduelle à point d'ébullition élevé,
dans lequel on effectue la séparation de l'ester brut, dans des unités de distillation qui sont mises en fonctionnement à une température de tête dans la zone allant de 100 à 220°C, à des pressions allant de 30 à 200 mbars et à une température de pot dans la zone allant de 180°C à 260°C,
caractérisé en ce que
les unités de distillation de l'ester brut sont pourvues d'empilages de structure qui ne contiennent essentiellement aucun élément structurel orienté horizontalement, et la différence respective de pression entre la tête de la colonne et le pot de colonne ne dépasse pas 30 mbar.

2. Procédé selon la revendication 1,
caractérisé en ce qu'
on fait fonctionner les unités de distillation à une température de tête de 100 à 220°C, à des pressions allant de 30 à 220 mbars, et à une température de pot de 180 à 245°C.

3. Procédé selon la revendication 2,
caractérisé en ce qu'
on fait fonctionner les unités de distillation à une température de tête de 100 à 220°C à des pressions allant de 30 à 200 mbars, et à une température de pot de 190 à 230°C.

4. Procédé selon au moins l'une des revendications 1 à 3,
caractérisé en ce que
la différence respective de pression entre la tête de colonne et le pot de la colonne ne dépasse pas 20 mbars.

5. Procédé selon la revendication 4,
caractérisé en ce que
la différence respective de pression entre la tête de la colonne et le pot de la colonne ne dépasse pas 15 mbars.

6. Procédé selon au moins l'une des revendications 1 à 5,
caractérisé en ce qu'
on effectue la distillation de l'ester brut dans un système à deux colonnes.

7. Procédé selon au moins l'une des revendications 1 à 6,
caractérisé en ce que
la distillation de l'ester brut est effectuée dans un système qui consiste pour l'essentiel en une chambre flash et une colonne post-connectée.

8. Procédé selon au moins l'une des revendications 1 à 7,
caractérisé en ce qu'
on met en oeuvre des empilages de structure en aciers inoxydables ou en d'autres matériaux résistants à la corrosion.

9. Procédé selon la revendication 8,
caractérisé en ce qu'
on met en oeuvre des empilages de structure en aluminium, ou en Hostelloy, ou en Titane, ou en plastique ou en céramique.

10. Procédé selon au moins l'une des revendications 1 à 9,
caractérisé en ce qu'
on met en oeuvre des empilages de structure ayant une surface de 100 à 800 m²/m³.
